Europäisches Patentamt

European Patent Office

Office européen des brevets

(11) Publication number: **0 216 472**
**A1**

(12) **EUROPEAN PATENT APPLICATION**

(21) Application number: 86306001.8

(22) Date of filing: 04.08.86

(51) Int. Cl.⁴: **C07C 1/04** , C07C 29/15 ,
B01J 27/04 , C01G 39/06

(30) Priority: 05.08.85 US 762239

(43) Date of publication of application:
01.04.87 Bulletin 87/14

(84) Designated Contracting States:
BE DE FR GB IT NL

(71) Applicant: THE DOW CHEMICAL COMPANY
2030 Dow Center Abbott Road P.O. Box 1967
Midland, MI 48640(US)

(72) Inventor: Stevens, Rex R.
4311 Andre Street
Midland Michigan 48640(US)

(74) Representative: Burford, Anthony Frederick et
al
W.H. Beck, Greener & Co. 7 Stone Buildings
Lincoln's Inn
London WC2A 3SZ(GB)

(54) **Attrition-resistant sulfides in syngas conversions.**

㊉ An attrition-resistant, unsupported catalytic sulfide containing Nb, Ta, Mo, W, Tc, or Re is used for preparing $C_{1-10}$ aliphatic hydrocarbons or $C_{1-10}$ mixed alcohols from synthesis gas. The catalytic sulfide is prepared by precipitating a centric particulate precursor, preferably $(NH_4)_2MoS_2O_2$, and then thermally decomposing the precursor. The catalytic sulfide can optionally have a metal promotor.

EP 0 216 472 A1

# ATTRITION-RESISTANT SULFIDES IN SYNGAS CONVERSIONS

This invention concerns an attrition-resistant, unsupported catalytic sulfide containing Nb, Ta, Mo, W, Tc or Re used to make $C_{1-10}$ aliphatic hydrocarbons or mixed alcohols from synthesis gas. The catalytic sulfide, which has a new crystal structure, is made by thermal decomposition of a centric particulate precursor.

Many examples of processes for converting synthesis gas into $C_{1-10}$ aliphatic hydrocarbons or alcohols are known. The following patents and publications are exemplary.

U.S. Patents 4,151,191 and 4,260,553 disclose methanation processes using unreduced molybdenum catalysts containing a lanthanide or actinide series metal.

U.S. Patents 4,151,190, 4,199,522 and 4,380,589 describe some Mo, W and/or Re catalysts useful for the Fischer-Tropsch production of hydrocarbons, but do not teach making commercially significant quantities of alcohols. These references note that hydrogen sulfide affects the activity of the catalyst.

U.S. Patent 4,177,202 teachs a process for making methane-rich hydrocarbons from $H_2/CO$ over a molybdena and optionally promoted cobalt or vanadium catalyst.. Selectivity to ethane is enhanced by the presence of hydrogen sulfide in the syngas feed.

U.S. Patent 2,490,488 discloses that molybdenum sulfide methanation catalysts acquire Fischer-Tropsch activity when promoted with an alkaline compound of an alkali metal. The example of the invention shows a 30 percent selectivity to $C_3+$ hydrocarbons and oxygenates. Of this 30 percent, no more than 44 percent boils near or above 65°C, the boiling point of methanol. Accordingly, the maximum possible alcohol selectivity is no more than 13.2 percent (44 percent of 30 percent).

R. B. Anderson et al., Industrial and Engineering Chemistry, Vol. 44, No. 10, pp. 24182424, disclose a number of catalysts containing zinc, copper, chromium, manganese, thorium, iron, occasionally promoted with alkali or other materials for making various alcohols. The authors state that ethanol is a major constituent, the yield of methanol is usually very small and a tentative summary of factors favoring the production of alcohols is high pressure, low temperature, high space velocity, high recycle ratio and carbon monoxide-rich synthesis gas.

Mills et al., Catalysis Rev., 8(2), 159-210 - (1973), review methanation and various supported and unsupported molybdenum and tungsten sulfide methanation catalysts including compositions with nickel and cobalt. Activity was moderate at best, and selectivities favored methane production from syngas.

Kruss, Justus Liebigs Annln. Chem., 225, 1-57 (1884), describes the preparation of thio-and oxythiomolybdates. The compounds therein were often prepared at lower temperatures. For example, ammonium oxythiomolybdate was prepared from aqueous mixtures at 6°C as fine prisms or needles.

Corleis, Justus Liebigs Annln. Chem., 232, 244-270 (1886), describes the preparation of thio-and oxythiotungstates. The compounds therein were often prepared from aqueous solution using hydrogen sulfide.

Prasad et al., J. Inorg. Nucl. Chem., 35, 1895-1904 (1973), teach the thermal decomposition of ammonium thio-and oxythiomolybdate and -tungstate.

U.S. Patents 4,243,553 and 4,243,554 teach the thermal decomposition of certain aliphatic-and heterocyclic-ammoniumthiomolybdate and -oxythiomolybdate powders into a high surface area molybdenum disulfide catalyst. The catalyst must be processed with binders, supports and/or metal doping agents such as tungsten and vanadium in order to retard sintering loss and thus to be useful in a fluid bed reactor.

U.S. Patent 4,459,369 teachs a catalyst for preparing oxygenates from syngas. The catalyst contains Cu and Ti, plus at least one other metal of Cr, Mn, Co, Mo, Rh, Pt or Fe, and an alkali or alkaline earth metal in stated ratios.

To make a commercially significant amount of $C_{1-10}$ aliphatic hydrocarbons or $C_{1-10}$ mixed alcohols, the process must employ a catalyst and conditions which are highly efficient. To be efficient, the catalyst must yield a high weight ratio of desired product per unit weight of catalyst in a given period of time. The catalyst must be stable and active for long periods of time between regenerations. This may be particularly difficult to accomplish, especially for alcohols, when the $H_2/CO$ ratio of the feed gas is low, such as less than 2 to 1. Ideally, the catalyst will be sulfur tolerant and will have a high selectivity to a commercial product to avoid purification or removal and disposal of by-products and to avoid separation into two or more product streams.

When the mixed alcohols product is to be used as a fuel replacement or a fuel additive, it may be desirable that the ratio of $C_1$ to $C_2$+ alcohols be no greater than a certain amount. As used herein, the ratio of $C_1$ to $C_2$+ alcohols means the molar ratio of methanol to $C_2$ to $C_5$ alcohols taken as a whole.

Excessive methanol is generally considered an unattractive additive to gasolines. Methanol may decrease drivability increase corrosion in the fuel system, and cause phase separation when used in excessive quantities. These problems can be alleviated by blending methanol with higher alcohols.

Accordingly, one may wish to synthesize mixed alcohols with no more than a certain amount of methanol in the blend. Consequently, one may wish to minimize the ratio of $C_1$ to $C_2$+ alcohols in mixed alcohols so that methanol may be purchased and blended into the mixed alcohols to give the maximum acceptable $C_1$ to $C_2$+ alcohols ratio.

In the light of the above, it would be desirable to enhance the efficiency of the overall commercial processes, for example, by decreasing the attrition of an unsupported catalyst in fluidized bed reactors which make the aliphatic hydrocarbons or mixed alcohols from synthesis gas.

Suprisingly, the present invention provides such an improved process for preparing $C_{1-10}$ aliphatic hydrocarbons or $C_{1-10}$ mixed alcohols which comprises reacting

a gaseous mixture of synthesis gas (which contains hydrogen and carbon monoxide) with a catalytic amount of an attrition-resistant, unsupported catalytic sulfide which contains Nb, Ta, Mo, W, Tc, or Re and which is prepared by precipitating a centric particulate precursor, then thermally decomposing the precursor.

Another embodiment of the invention is a centric particulate precursor comprising $(NH_4)_2Mos_2O_2$, $(NH_4)_2WS_4$, or $(NH_4)_2Mos_4$ and its thermal decomposition products.

The process of the present invention prepares well-known products useful as combustibles, solvents and, when appropriately treated, antiseptics. The process can be extremely efficient and adaptable, showing high conversion and variable selectivity, and can be especially efficient as a fluidized bed process. In particular, the unsupported catalytic sulfide is especially adaptable to a fluidized bed process wherein the catalyst preferably shows surprising attrition-resistance and high activity.

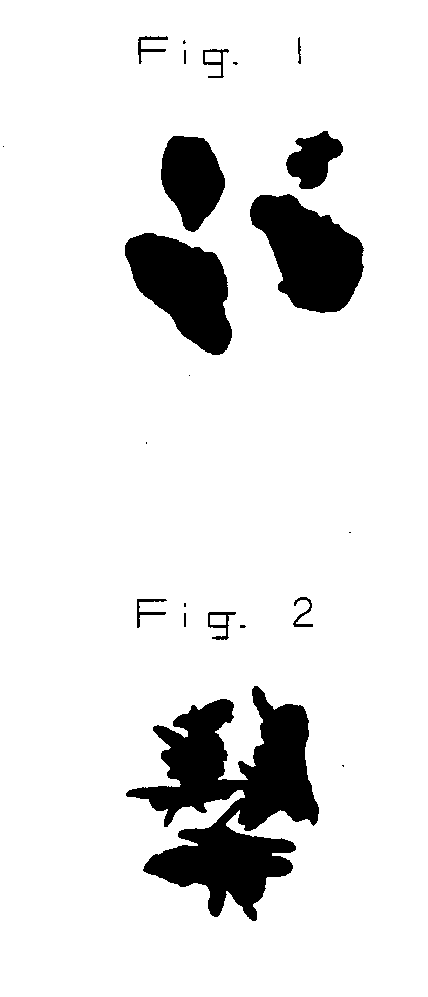

In the accompanying drawings, Figure 1 generally illustrates the centric precursor $(NH_4)_2$-$Mos_2O_2$; and Figure 2 generally illustrates the prior art needlelike precursor $(NH_4)_2Mos_2O_2$.

The catalytic sulfide contains a catalyst metal of Nb, Ta, Mo, W, Tc, or Re. Mo and W are preferred, and Mo is most preferred.

The catalytic sulfide may be present in the catalyst in any amount that demonstrates catalytic activity in the process in which it is used. Preferably, the catalytic sulfide is present in percentages based on the weight of the total catalyst, of from 2 to 98 percent, more preferably from 5 to 95 percent, most preferably from 10 to 90 percent, and most particularly preferred from 20 to 80 percent.

The term sulfide in catalytic sulfide herein means the catalyst metal may be generally present as the sulfide. It is not necessary that any particular stoichiometric amount of sulfide be present, but only that the catalyst metal is present in combination with sulfur. Some of the catalyst metal may be present in combination with other elements, for example oxygen as oxysulfides. The free metal may possibly be present if reduction of the composition has ensued during or after thermal decomposition of the precursor. Such reduction could be by flowing, hot hydrogen.

Preferably, the range of the stoichiometric ratio of sulfur to the catalyst metal in the catalytic sulfide is from 0.1 to 3, more preferably from 1.8 to 2.3. Most preferably, the catalytic sulfide is molybdenum or tungsten disulfide.

Catalytic herein means the sulfide has catalytic activity of the described character. For example, the catalytic activity may be conversion of synthesis gas in the presence of a catalyst metal sulfide into $C_{1-5}$ aliphatic hydrocarbons at a conversion percent of 12 or more. The catalyst metal sulfide would be a catalytic sulfide therein.

The catalytic sulfide is made from thermal decomposit6ion of a centric particulate precursor. The centric particulate precursor itself contains sulfur as a component. Examples of this precursor are centric $(NH_4)_2Mos_4$, $(NH_4)_2WS_4$ and $(NH_4)_2Mos_2O_2$, with $(NH_4)_2MosO_2$ being preferred.

As used herein, centric means having mass concentrated about a center. Examples of centric shapes of precursors include the following: spheres, tetrahedrons, slightly to moderately distorted tetrahedrons, cubes, square antiprisms, dodecahedrons, octahedrons, slightly to moderately tetragonally, rohombically and trigonally distorted octahedrons, ellipsoids having foci within a distance of 10 percent of the length of the major axis, square prisms having a height about two times the length of the base, and square based pyramids of a height about $1\frac{1}{2}$ times the length of a side of the base. Examples of shapes not considered centric include square prisms having a height four or more times the length of the base. Thus, leaflets, needles, needle-like chemical solids, and needle agglomerates such as would be represented by a spiked mace shape, are not considered centric.

Preferable centric shapes have about 20 percent or more volume included within the boundary established by a sphere of a center which is also a geometrical center of a generally representative sample shape of the particulate precursor. The radius of the sphere is established by the nearest boundary of the representative particular precursor. More preferably, the included volume is about 35 percent or more and most preferably about 45 percent or more. It is especially preferred that any appendages on the basic centric shape include about 10 percent or less of the total volume of the basic shape and more especially about 5 percent or less. A basic centric shape is a generally representative sample shape of the particulate precursor which is centric.

Particulate herein means composed of discrete particles of sufficient independent size which are considered solids at the temperature and pressure of thermal decomposition and in the reaction as a catalyst. For example, discrete particles of molybdenum disulfide are generally considered solid and particulate. Powders are not composed of discrete particles of sufficient independent size to be considered particulate.

Generally, the resultant thermally decomposed catalytic sulfide from the centric particulate precursor has a higher attrition-resistance in a fluidized suspension than has a comparable thermally decomposed catalytic sulfide from a particulate precursor which is not considered centric, such as needles. Preferably, the higher attrition-resistance is measured under regular test conditions.

Regular test conditions herein include the measurement of 10.0 g of sample consisting of the catalytic sulfide in an inert gas consisting of dry nitrogen at about 25°C and about one atmosphere pressure. The vessel holding the suspended particles is a smooth-walled glass cylinder of 12-inch - (30.5 cm) height or greater and one-inch (2.5 cm) internal diameter, with the $C\infty$ axis of symmetry of the cylinder mounted in vertical alignment with the force of earth's gravity. The lower end cap of the glass cylinder is of fritted glass, the upper end is generally open and allows unrestricted nitrogen flow. The fluid bed suspension is maintained for a period of thirty days by continuously maintaining a Gas Hourly Space Velocity (i.e., GHSV) of the nitrogen through the lower end cap at a rate sufficient to maintain the fluidized suspension, excluding attrited carryover dust and the like, at a volume about two or three times the volume of the 10.0 g sample in the cylinder before the fluidization. The other conditions are otherwise normal (e.g., no microwave bombardment or sound bombardment).

Under regular test conditions, the higher attrition-resistance of the catalytic sulfide is preferably measured by a percent loss of catalytic sulfide of about 33 percent by weight or less. More preferably, the loss is about 15 percent by weight or less and most preferably about 5 percent by weight or less.

The particle size of the precursor is such that its thermal decomposition results in a catalytically useful catalytic sulfide. Generally, the particle size of the centric particulate precursor is from 5 to 1000 $\mu$m. More preferably, lower limits of the range of the particle size of the centric precursor, found by measurement of the longest line segment traversing the internal space of a reasonably representative sample shape of the centric precursor, are about 10 $\mu$m , preferably about 20 microns, more preferably about 37 $\mu$m and most preferably about 50 $\mu$m. Equivalent more preferable upper limits of the range are about 1000 $\mu$m, most preferably about 300 $\mu$m.

The particle size of the centric precursor affects the catalytic sulfide. Preferably, thermal decomposition of the centric particulate precursor results in a non-agglomerated particulate catalytic sulfide. Non-agglomerated herein means the particles substantially do not stick together as a fused mass.

Preferable particle sizes of the catalytic sulfide are those which can advantageously be used in fluidized bed reactors. Generally, the particle size of the catalytic sulfide is from 5 to 300 $\mu$m. Preferably, the particle size limits of the particle size range, measured in the same manner as the centric precursor, are from 10 to 300 $\mu$m, preferably from 20 to 200 $\mu$m, more preferably from 37 to 150 $\mu$m, and most preferably from 50 to 150 $\mu$m.

The particles may be advantageously screened anytime during or after processing to obtain particle size distribution advantageous for particular applications. Screening is, however, often not necessary. For example, it may be desired to have particles of from 70 to 100 $\mu$m in a fluidized bed to convert syngas. The centric particulate precursor may be made to conform to these requirements, or the precursor or the catalytic sulfide may be screened to conform to them.

The centric particulate precursor is made by controlled precipitation, forming particles of the desired size and shape. Preferably, the precipitation is from a liquid mixture. Control of the precipitation is achieved by control the nucleation and/or crystal growth of the centric particulate precursor. As a rule, the number of particles and, therefore, the particle size of the precursor, are determined by the number of nuclei formed in the nucleation. The number of nuclei formed, and hence control of nucleation, is approximately related to the relative supersaturation of the liquid mixture.

In general, the larger the relative supersaturation, the smaller will be the size of the individual particles of the centric precursor.

Relative supersaturation is the ratio of (Q-S)/S. Therein, Q is the actual concentration of a dissolved precursor at the instant precipitation begins, and S is the equilibrium concentration of solute in a saturated solution. Stirring of the liquid mixture lowers the relative supersaturation. Larger crystals are also often induced by more dilute solutions at the time of the beginning of precipitation.

If the nucleation is induced, as is very likely the case, suitable solid particles may be present or introduced that provide sites upon which crystal growth of the precursor can be initiated. Suitable particles include added finely divided powders of the centric precursor to the catalytic sulfide, smaller particles of the centric precursor, particles of catalytic sulfide too small to be effectively employed in a fluidized bed reactor application, particulate promoters, inert supportive particles smaller than the desired size of the centric precursor, and other supports smaller than the desired size of the centric precursor such as alumina, titania, zinc and iron oxides, carbon and the like. Reagent-grade chemicals themselves typically provide extremely large numbers of nucleation sites as they may contain 0.005 to 0.01 percent insoluble matter. Preferably, nucleation is not induced by the addition of the inert supportive particles and the other supports smaller than the desired site of the centric precursor. Spontaneous nucleation may also occur.

Although other compounds may be added, their addition may significantly reduce the size of the centric particulate precursor, especially if present during the early stages of crystal growth such as nucleation. For example, early addition of hydrogen sulfide typically results in powdered ammonium-and alkylammoniumthio-and oxythiomolybdates. These powders are unusable in fluidized bed applications after thermal decomposition unless processed with binders and the like.

The choice of liquid solvent has a large effect on the size and shape of the centric particulate precursor. Water and $C_{1-4}$ hydroxy-containing hydrocarbons such as methanol, ethanol, ethylene glycol, isopropanol, n-propanol, glycerol, isobutanol, n-butanol, sec-butanol, t-butanol and the like are preferred solvents. Water is most preferred.

It may be advantageous to use one or more solvents in conjunction with others to aid in precipitation of the centric particulate precursor. For example, an alcohol like ethanol may be added to water or solvent mixtures may be used to aid in such precipitation.

The pH of the solution often has a marked effect on the formation of the centric particulate precursor. Preferably, the aqueous pH is about seven or greater.

Generally, slow growth of the nuclei of the centric particulate precursor is advantageous. The growth of the centric particulate precursor is most easily controlled by regulating the temperature of the liquid mixture forming the centric particulate precursor. Temperatures from 0 to 40°C are preferred, more preferably from 10 to 25°C and most preferably from 10 to 20°C. Temperatures between 12 to 18°C are especially preferred, for example, in producing centric $(NH_4)_2MoS_2O_2$ (from aqueous mixtures.

Centric $(NH_4)_2MoS_2O_2$ is especially preferred as the catalytic sulfide. Centric particulate precursors based on centric $(NH_4)_2MoS_2O_2$ are also especially preferred.

The centric particulate precursor can be removed from the liquid mixture at the desired size by methods known in the art such as straining through a mesh, filtration, scooping, density gradient centrifugation, liquid suspension techniques and the like. After removal from the liquid mixture, the centric particulate precursor may be dried. Drying in an inert atmosphere, such as dry nitrogen or dry argon, at temperatures about 80°C or below, preferably about 50°C or below, and most preferably about 30°C or below.

At any time during processing, it may be advantageous to add another catalyst metal (or added metal promoter) to the centric particulate precursor at hand. The centric particulate precursor must at some time be thermally decomposed at least once.

Thermal decomposition follows the formation of the centric particulate precursor. Thermal decomposition is such that the centric particulate precursor changes its composition. The composition change preferably enhances catalytic activity (and may even be the basis for the catalytic activity). The composition change may be effected by any added promoters and by the method of thermal decomposition employed. Typically, the physical shape and size of the precursor is generally retained after thermal decomposition.

Thermal decomposition is accomplished by methods such as bombardment with infrared radiation, microwave radiation, or through heating in an inert liquid or in an inert atmosphere. Heating in an inert atmosphere is preferred. Suitable temperatures for heating are from 200 to 1200°C, preferably from 400 to 700°C.

Times of thermal decomposition are such that the required, and desired level of, decomposition occur. Usually, the longer the thermal decomposition occurs, the more decomposed the centric particulate precursor becomes. Preferably, thermal decomposition is maintained for from $\frac{1}{2}$ to 2 hours and more preferably from 1 to 2 hours.

Pressures during the thermal decomposition are such that the centric particulate precursor is decomposed. Lower and higher pressures during thermal decomposition may affect the rate, level of decomposition and/or composition of the decomposed precursor. Preferably, the pressure during decomposition is about ambient atmospheric pressure or below, more preferably about ambient atmospheric.

Unsupported catalytic sulfides from centric - $(NH_4)_2MoS_2O_2$ are especially preferred. Unsupported catalytic sulfides based on centric $(NH_4)_2MoS_2O_2$ are also especially preferred. The centric, thermally decomposed product catalyst is preferably $MoS_2$. Other centric, thermally decomposed catalysts are the sulfides of Nb, Ta, W, Tc or Re.

The thermally decomposed precursor may be optionally treated with a reactive atmosphere, for example air, oxygen, carbon monoxide, methane, hydrogen and hydrogen sulfide, preferably hydrogen and hydrogen sulfide and more preferably in combination as a sulfiding atmosphere. Temperatures of treatment are generally equivalent to those of thermal decomposition. Times may be longer, such as for 4 hours or more. Higher pressures may be more advantageous, such as about ambient atmospheric pressure or greater.

The catalytic sulfide may optionally contain a metal promoter, including the following types: another transition metal; a lanthanide or actinide series metal; an alkali or alkaline earth metal; or combination thereof, in free or combined form. In free or combined form means the metal component is present as a metal, alloy, compound or adduct thereof. The transition metals herein do not include Cd and Hg, but do include Zn. Preferred transition metal promoters are Fe, Ni and Co compounds, more preferably as their sulfides. It is most preferred that the Fe, Ni and Co contain ferrous, nickelous or cobaltous sulfides. Cobalt compounds are especially preferred. The sulfide in the finished catalyst may be obtained by combination with the existing sulfide or may often be produced by conversion from another Fe, Ni or Co compound by sulfiding. The sulfided transition metal compound-containing catalysts, especially cobalt, are preferably protected from air or other oxidizing atmosphere before use.

Preferably, sulfiding is accomplished by reactive treatment with hydrogen sulfide gas at elevated temperatures, more preferably the hydrogen sulfide is mixed with hydrogen gas, most preferably at a level of from 1 to 10 percent by weight. Preferable elevated temperature limits are those herein articulated for the thermal decomposition and the reactive atmospheric treatments.

The more preferred transition metal promotors may especially be employed in the catalytic sulfide in a process for making mixed alcohols to shift the selectivity from the production of preponderantly methanol to $C_2+$ alcohols from synthesis gas. Such a shift may be significantly enhanced by the addition or recycle of methanol to the synthesis gas feed.

Together, the lanthanide and actinide series metals are herein considered one type. The lanthanide series metals include lanthanum and the elements of atomic number 58 through 71, inclusive. The actinide series metals include actinium and the elements of atomic number 90 through 103, inclusive. Preferred lanthanide and actinide series promoters are La, Ce, Sm and Th, with La and Th more preferred and Th most preferred.

The lanthanide and actinide series metals, especially the preferred ones, may enhance the activity of the catalytic sulfide in the conversion of synthesis gas into $C_{1-10}$, and especially into $C_{1-5}$, aliphatic hydrocarbons. The more preferred lanthanide and actinide series metals may also be employed as Fischer-Tropsch promoters or in special conjunction therewith. Reduction of the catalytic sulfide containing the lanthanides or actinides may enhance activity. Reduction may be accomplished by methods known in the art. For example, reduction by contact with a liquid phase reducing agent such as hydrazine or hydroxylamine or reduction using a metal hydride may be advantageous. Reduction at elevated temperatures with a reactive reducing atmosphere, such as methane, hydrogen, hydrogen sulfide, and mixtures of these may be advantageous and is preferred. Hydrogen and hydrogen sulfide are more preferred reactive reducing atmospheres. The conditions for reactive atmosphere reduction are as herein generally described for the optional treatment with a reactive atmosphere.

Together, the alkali and alkaline earth metals are herein also considered one type. The alkali and alkaline earth metals are also preferred promoters and are among the Fischer-Tropsch promoters, for example Zn, Ca, Mg, K or Na. The alkali metals are more preferred, and potassium is most preferred.

The Fischer-Tropsch promoters enhance the formation of mixed alcohols from synthesis gas. Excess amounts of a Fischer-Tropsch promoter of an alkali or alkaline earth metal may decrease activity.

The total amount of metal promoter of any type may be present at any level which enhances the production of the desired product. Preferred total levels of promoter are from 0.05 to 30 weight percent as if a free element in the finished catalyst, more preferably from 0.1 to 20 weight percent and most preferably from 1 to 20 weight percent.

Similarly, preferred levels of each type of promoter are 0.05 to 20 weight percent as if a free element in the finished catalyst, more preferably from 0.1 to 10 weight percent and most preferably from 1 to 7 weight percent.

The promoter may be added at any time during or after the preparation of the catalytic sulfide and preferably after formation of the catalytic sulfide. It may be added as an ingredient in another promoter, the catalytic sulfide component or other material. The promoter may be added by physical mixing, solution impregnation or in forming the centric particulate precursor. For example, carbon material prepared from coconut shells often contains small amounts of alkali metal oxides or hydroxides; the centric precursor may have its crystallization induced by the addition of such a particulate material.

Based upon the weight of the total catalyst, any additional material generally considered a support when present generally comprises an amount about 5 percent or less of the finished catalyst. Additional refers to materials other than that of a catalyst metal which is made into a centric particulate precursor. When such additional material is present in such amounts about 5 percent or less, the catalytic sulfide is herein considered to be unsupported. Preferably, any additional material generally considered a support is present in an amount about 2 percent or less of the finished catalyst. More preferably, no such additional material is added.

Examples of materials generally considered supports include: the aluminas, basic oxides, the silicas, carbons, or suitable solid compounds of magnesium, calcium, strontium, barium, scandium, yttrium, lanthanum and the rare earths, titanium, zirconium, hafnium, vanadium, thorium, uranium, and zinc, of which oxides are exemplary compounds.

The catalysts of the invention may be employed individually or in combination with other catalysts. In combination with other catalysts, these catalysts may tend to progressively modify the usual effects in accordance with their individual characteristics so that quantitatively intermediate results may be achieved. For example, the catalysts of the present invention may be combined with typical hydrogenation catalysts such as cobalt and nickel.

Under preferred reaction conditions the catalyst is stable for long periods of time. Activity and selectivity are preferably substantially retained after 700 hours of operation, more preferably after 2000 hours and most preferably after 4000 hours operation. Under ideal conditions it may be stable and active for as many as 6000 hours or more.

The finished catalyst may be used in a fixed bed, moving bed, fluid bed, ebulliated bed or a graded bed wherein concentration and/or activity of the catalyst varies from inlet to outlet in similar manner to known catalysts. Because of the high attrition-resistance of the catalytic sulfide, it may find preferred use in fluidized bed reactors.

The catalytic sulfide preferably shows high activity in the conversion of synthesis gas into the desired product under reactive conditions. Reactive conditions are those known in the art or herein, such as generally illustrated in the example and especially wherein the synthesis gas consists of about 95 mole percent hydrogen and carbon monoxide gases at an $H_2/CO$ ratio of about 1.0 and about 5 mole percent dry nitrogen, GHSV corrected to standard temperature and pressure (i.e., STP) about 1000 hours$^{-1}$ or slightly less, temperature about 300°C and pressure about 500 psig (3.5 MPa), with no recycle.

This surprising activity is preferably about $5 \times 10^7$ units or above, more preferably about $8 \times 10^7$ units or above, most preferably about $1 \times 10^8$ units or above, and especially about $3 \times 10^8$ units or above, wherein activity as defined herein is as generally defined by Anderson etal., Ind. Eng. Chem., 44 (2), 391-401 (1952). In particular,

$$\text{Activity} = -\text{GHSV} \ \frac{\ln(1-C)}{P} \ e^{E/RT}$$

wherein

GHSV is the gas hourly space velocity in units of hour$^{-1}$ as if at STP;

ln is the natural logarithm of the quantity (1-C);

C is the fraction of synthesis gas converted into products excluding carbon dioxide;

E is the activation energy (e.g., 18,100 cal/mole - (75.8 kJ/mole) for the process of converting the synthesis gas into $C_{2-5}$ hydrocarbons);

R is the gas constant (i.e., e.g., 1.987 cal/mole °K; 8.314 J/mole °K);

T is the temperature in degrees Kelvin (i.e., °K); and

P is the pressure of the feed synthesis gas in units of atmospheres.

By percent $CO_2$-free carbon selectivity it is meant the percent of carbon in a specific product with respect to the total carbon converted from carbon monoxide to some product other than carbon dioxide. For example, one mole of ethyl alcohol is 2 moles of carbon and would represent 50 carbon mole percent selectivity to ethanol if 4 moles of CO were converted to products other than $CO_2$.

The hydrogen and carbon monoxide required for this process can be obtained by methods known in the art. Examples are gasification of hydrocarbonaceous materials such as coal, high specific gravity oils, or natural gas; as a by-product of partial combustion cracking of hydrocarbons; by steam reforming of liquid or gaseous hydrocarbons; through the water-gas shift reaction; or some combination of these. The two components may also be generated separately and combined for the subject reaction.

The molar ratio of hydrogen to carbon monoxide in the feed gas which contacts the catalyst is such that the $C_{1-10}$ aliphatic hydrocarbons or mixed alcohols are produced. Preferably, limits of the ratio are from 0.25 to 100, more preferably from 0.5 to 5, and most preferably from 0.7 to 3. Preferably, some hydrogen sulfide is also in the feed gas, preferably, from 10 to 200 ppm, most preferably from 20 to 100 ppm.

A desired product may comprise a mixture of $C_{1-10}$ aliphatic hydrocarbons and mixed alcohols. Preferably, the desired product either comprises a mixture of $C_{1-10}$ aliphatic hydrocarbons or comprises a mixture of $C_{1-10}$ mixed alcohols. The desired product may more preferably either comprise a mixture of methane (i.e., methanation process) or comprise a mixture of $C_{2-10}$ aliphatic hydrocarbons (i.e., Fischer-Tropsch process for production of aliphatic hydrocarbons of up to ten carbons per molecule). A most preferably desired hydrocarbon product is $C_{2-5}$ aliphatic hydrocarbons and especially $C_{2-5}$ alkanes. Of the mixed alcohols product, $C_{1-5}$ mixed alcohols are more preferred, and $C_{2-5}$ mixed alcohols are most preferred. The mixed alcohols are preferred over the aliphatic hydrocarbons.

Pressures are such that the $C_{1-10}$ aliphatic hydrocarbons or mixed alcohols are produced. Generally, the selectivity to the desired product can be enhanced by varying the pressure.

In the normal operating ranges, the higher the pressure at a given temperature, the more selective the process will be to mixed alcohols. The minimum preferred pressure for mixed alcohols production is about 500 psig (3.5 MPa). The more preferred minimum for mixed alcohols production is about 750 psig (5 MPa) with about 1,000 psig (7 MPa) being a most preferred minimum. While 1,500 psig (10 MPa) to 4,000 psig (28 MPa) is the most desirable range, higher pressures may be used and are limited primarily by cost of the high pressure vessels, compressors and energy costs needed to carry out the higher pressure reactions. About 10,000 psig (69 MPa) is a typical preferred maximum with about 5,000 psig ( 35 MPa ) a more preferred maximum. About 3,000 psig (21 MPa) is a most preferred maximum pressure.

Methane production is generally favored by generally lower pressures. Preferably, pressures such as from 100 psig to 2000 psig (about 800 kPa to about 14 MPa) are employed.

Fischer-Tropsch processes for the production of $C_{2-10}$ aliphatic hydrocarbons, especially for the production of $C_{2-5}$ aliphatic hydrocarbons, are favored by generally lower pressures such as from atmospheric to about 1500 psig (gauge pressure of about 10 MPa ). Pressures therein are preferably from 150 to 700 psig (gauge pressure from 1,000 to 4,800 kPa) and more preferably are from 200 to 500 psig (gauge pressure from 1,400 to 3,500 kPa).

Temperatures are such that the $C_{1-10}$ aliphatic hydrocarbons or mixed alcohols are produced. The selectivity to the desired product is also a function of temperature and is interrelated with the pressure function. However, the minimum temperature used is governed by productivity considerations and the fact that at temperatures below about 200°C, volatile catalytic metal carbonyls may form.

Accordingly, for mixed alcohols production, the preferred temperature is generally from 200 to 400°C. A more preferred maximum is about 350°C. However, the most preferred range of operation is from 240 to 325°C.

Methane production is favored by generally higher temperatures. Preferably, temperatures from 300 to 600°C are employed therein.

Fischer-Tropsch processes for the production of $C_{2-10}$ aliphatic hydrocarbons, especially for the production of $C_{2-5}$ aliphatic hydrocarbons, are favored generally by temperatures from 200 to 500°C. Preferably, temperatures from 300 to 420°C are employed therein.

Alcohols herein are compounds otherwise hydrocarbons which contain at least one hydroxy functionality bound to carbon therein. Alcohols do not include other hydroxy-containing or oxygenated hydrocarbon compounds. For example, the methoxy portion of methyl acetate is not counted as methanol. Salicylaldehyde is not an alcohol herein only because of its aldehyde functionality. The mixed alcohol fraction usually formed in the mixed alcohols synthesis may contain methanol, ethanol, 1-propanol, 2-propanol, 1-butanol, 2-butanol, 2-methyl-1-propanol, 2-methyl-2-propanol, other $C_{2-4}$ alcohols and $C_{5-10}$ alcohols. Preferably the selectivity to $C_{2-5}$ alcohols is high while the selectivity to methanol is low. The product mixture, as formed under preferred conditions, contains only small portions of other oxygenated compounds besides alcohols. These other compounds may not be deleterious to using the product, as is, in motor fuels.

Preferably, the mixed alcohols fraction is formed in at least about 50 percent $CO_2$-free carbon selectivity and especially greater than about 70 percent. Under the most preferred conditions, total alcohols may be obtained in up to about an 87 percent $CO_2$-free carbon selectivity.

Preferably, $CO_2$-free carbon selectivity to methanol is less than about one-half the $CO_2$-free carbon selectivity to the $C_{1-10}$ mixed alcohol fraction, more preferably about one-third or less and most preferably about one-fifth or less and especially about one-tenth or less. It is especially preferred that the methanol $CO_2$-free carbon selectivity is similarly compared with $C_{1-5}$ mixed alcohols $CO_2$-free carbon selectivity. As conversion increases, the product distribution of mixed alcohols produced usually shifts toward higher molecular weight alcohols.

Preferably, the co-products formed with the alcohol fraction in the mixed alcohols process are primarily gaseous products. That is, they are preferably primarily $C_{1-4}$ hydrocarbons. Preferably, $C_5+$ hydrocarbons are coproduced therein at less than about 20 percent $CO_2$-free carbon selectivity, more preferably at less than 10 percent and most preferably at less than 5 percent. Lower amounts of normally liquid hydrocarbons make the normally liquid alcohols easier to separate from by-products.

Under preferred conditions, the amount of water formed is sucstantially less than the amount of desired product formed. Typically there is less than about 20 weight precent water based on the quantity of desired product, especially wherein the desired product is $C_{1-10}$, more especially $C_{1-5}$ and most especially $C_{2-5}$, mixed alcohols. Preferably this is less than about 10 weight percent and most preferably about 5 weight percent or less. This water may be removed by known techniques. For example, in a mixed alchols process, if the water content is about 2 weight percent or less based on mixed alcohols, the water may advantageously be removed by absorption on molecular sieves. At higher water contents, one may use a water gas shift drying step as disclosed in British Patents 2,076,015 and 2,076,423. Use of a sulfur tolerant catalyst such as Haldor Topsoe SSK is preferred in the water gas shift drying step.

The $H_2/CO$ gas hourly space velocity GHSV is a measure of the volume of hydrogen plus carbon monoxide gas at standard temperature and pressure passing a given volume of catalyst in an hour's time. GHSV is such that the $C_{1-10}$ aliphatic hydrocarbons or mixed alcohols are produced. Preferably, lower limits of GHSV are about 100 hour$^{-1}$ and more preferably about 300 hour$^{-1}$. Preferably, equivalent upper limits are about 20,000 hour$^{-1}$ and more preferably about 1,000 hour$^{-1}$ for methane, about 3,000 hour$^{-1}$ for $C_{2-10}$ and especially $_{2-5}$ aliphatic hydrocarbons and about 5,000 hour$^{-1}$ for the mixed alcohols. Selectivity to the $C_{1-10}$ aliphatic hydrocarbons or mixed alcohols usually increases as the space velocity decreases. Conversion of carbon monoxide typically decreases as space velocity increases.

Preferably, at least a portion of the unconverted hydrogen and carbon monoxide in the effluent gas from the reaction, more preferably after removal of the desired product, water and carbon dioxide formed and even more preferably any other by-products formed, may be recycled to the reaction. For example, in a $C_{1-10}$ mixed alcohols process, other by-products formed may include hydrocarbons.

The amount of recycle is expressed as the recycle ratio which is the ratio of moles of gases in the recycle stream to the moles of gases in the fresh feed stream. A recycle ratio of zero is within the scope of the invention with at least some recycle preferred. A recycle ratio of at least about one is more preferred and at least about three is most preferred.

The synthesis should be carried out at as little feed conversion per pass as is compatible with economic constraints related to the separation of the desired product from unreacted feed and other by-product gases. Preferably, the space velocity and recycle ratios are adjusted to obtain about 15-25 percent conversion per pass.

The following example is illustrative of the invention, but not the only possible embodiment.

In the example, the reagents are of reagent-grade, ot higher, quality. Catalytic-grade reagents are reagents of such higher quality than reagent-grade and may be advantageously employed in the invention.

Example

An unsupported catalytic sulfide with suitable particle size and shape for use in a fluid bed reactor is prepared as follows:

16 g of ammonium paramolybdate (90.6 percent $MoO_3$) is dissolved in 85 ml of $H_2O$ and 15 ml of concentrated $NH_4OH$. The solution is maintained at about 15°C and stirred vigorously as 65 g of 22 percent $(NH_4)_2S$ solution is added dropwise over a 1-hour period. After 2/3 of the $(NH_4)_2S$ solution has been added, large orange crystalls, which include substanial amounts of $(NH_4)_2MoS_2O_2$, begin to form.

After addition of $(NH_4)_2S$ is complete, the slurry is filtered, and the crystals are dried overnight at 25°C in flowing $N_2$. The dry product is generally composed of centric-shaped crystals with a general particle size distribution of 25-200μm. The dry crystals are heated in flowing $N_2$ at 500°C for 1 hour.

A 6.6-g portion of the resulting black catalytic sulfide, sucstantially of $MoS_2$ (screened to +170-100 mesh; >88μm to <150 μm is slurried in a solution of 0.8 g KOH dissolved in 20 ml of $CH_3OH$ and allowed to evaporate to dryness in air. The alkalized $MoS_2$ (6.5 g, 5.5 cm³) is mixed with 5.5 cm³ of tabular $Al_2O_3$, loaded between layers of tabular $Al_2O_3$ filler in a heated $\frac{1}{2}$ in. × 3 ft. (1.3 cm × 0.9 m) (internal dimensions) stainless steel reactor, and exposed to syngas consisting of 95 mole percent hydrogen and carbon monoxide and 5 mole percent dry nitrogen under the following conditions:

Temperature (°C)±　　　　　　227
Pressure (psig)　　　　　　　1500 (10 MPa)
$H_2$/CO (molar ratio)　　　1.01
GHSV (hours$^{-1}$)　　　　　1115
Recycle ratio　　　　　　　　0

The following is observed:

CO Conversion (%)　　　　　　13.8
Lb CO converted per lb (0.45 kg)
　of catalyst per hour　　　0.12
$CO_2$ produced (%)　　　　　22.5
　(% based on moles $CO_2$
　formed/mole CO converted)

$CO_2$ Free Selectivities (%)
　GAS PHASE
　　$CH_4$　　　　　　　　　　11.8
　　$C_2$+ hydrocarbons　　　 2.4

　Subtotal　　　　　　　　　 14.2

　LIQUID PHASE
　　Methanol　　　　　　　　　43.0
　　Ethanol　　　　　　　　　 26.0
　　Propanols　　　　　　　　　6.9
　　Butanols　　　　　　　　　 1.8
　　Pentanols　　　　　　　　 　0

　Subtotal　　　　　　　　　 77.7

　Other oxygenates
　　and hydrocarbons　　　　　 8.1
　　(assuming $C_4$ oxygenates)

$H_2O$ (wt % of liquid phase)　　 1.6

To demonstrate the attrition-resistance of the catalytic sulfide, a 10.0 g portion of +170-100 mesh (>88 μm td <150 μm) $MoS_2$, non-alkalized as prepared above, is fluidized under regular conditions in a 12-inch height by 1-inch diameter (30.5 cm ˣ 2.5 cm) glass cylinder using $N_2$ at 25°C and about one atmosphere pressure. After 30 days of continuous fluidization, 9.6 g of $MoS_2$ catalyst is recovered, indicating a 4 percent attrition loss in 1 month. Accompanying Figure 1 shows the microscopic crystal structure of the resulting precursor catalyst of this invention, $(NH_4)_2MoS_2O_2$. The centric structure is clearly evident. The drawing is taken from a photomicrograph using no greater than ˣ 200 magnification with polarized light, and the crystals vary from 50 to 250 μ.

This above result compares to a 45 percent attrition loss in 2 weeks for non-alkalized $MoS_2$, composed of needles or needle agglomerates, under otherwise comparable conditions. Accompanying Figure 2 shows the microscopic crystal structure of the prior art precursor catalyst. The precursor catalyst was prepared as described by Gerhard Krüss in Justus Liebig's Amalender Chemie 225, 1-57 (1884). The drawing was obtained in a similar manner to Figure 1 and the crystals are from 50 to 250 μm.

## Claims

1. A process for preparing $C_{1-10}$ aliphatic hydrocarbons or $C_{1-10}$ mixed alcohols which comprises reacting a gaseous mixture containing hydrogen and carbon monoxide with a catalytic amount of an unsupported catalytic sulfide characterised in that the catalytic sulfide is attrition-resistant, and contains Nb, Ta, Mo, W, Tc or Re, and is prepared by precipitating a centric particulate precursor and then thermally decomposing the precursor.

2. A process as claimed in Claim 1, wherein the catalytic sulfide additionally contains a metal promoter, in free or combined form, the metal promoter being selected from Fe, Ni, Co, La, Ce, Sm, Th, Na, K, Zn, Mg and Ca.

3. A process as claimed in Claim 1 or Claim 2, wherein the $C_{1-10}$ mixed alcohols or $C_{1-10}$ aliphatic hydrocarbons are produced using a fluid bed reactor.

4. A process as claimed in any one of the preceding claims, wherein the catalytic sulfide has an attrition-resistance of 33 percent by weight or less as measured by a loss of catalytic sulfide.

5. A process as claimed in any one of the preceding claims, wherein the catalytic sulfide contains Mo or W.

6. A process as claimed in Claim 5, wherein the precursor is $(NH_4)_2 MoS_2O_2$.

7. A process as claimed in Claim 6, wherein the precursor is precipitated from an aqueous solution at a temperature from 12 to 18°C, then thermally decomposed in nitrogen at a temperature from 400 to 700°C for 0.5 to 2 hours.

8. A process as claimed in Claims 5 to 7, wherein the catalytic sulfide contains Mo and a metal promoter containing Co and an alkali metal.

9. The process as claimed in any one of the preceding claims, wherein the aliphatic hydrocarbons produced are $C_{2-5}$ alkanes.

10. A process as claimed in any one of Claims 1 to 8, wherein the hydrocarbon produced is methane.

11. A process as claimed in any one of the preceding claims, wherein the mixed alcohols produced are $C_{1-5}$ mixed alcohols and are produced in at least 70 percent $CO_2$ free carbon selectivity.

12. A process as claimed in any one of the preceding claims, wherein the catalytic sulfide has an activity of $1 \times 10^2$ units or greater.

13. A precursor catalytic composition of centric $(NH_4)_2MoS_2O_2$.

14. A composition as claimed in Claim 13, which is thermally decomposed to yield centric $MoS_2$.

15. A composition as claimed in Claim 14, which contains a metal promoter as defined in Claim 2.

16. A centric particulate precursor catalyst comprising $(NH_4)MoS_2O_2$, $(NH_4)_2WS_4$, or $(NH_4)_2MoS_4$ and/or its thermal decomposition products.

Fig. 1

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | US-A-4 243 554 (NAUMANN et al.)<br><br>* Claims; column 9, lines 23-31; summary *<br><br>--- | 1,5,6,<br>10,13,<br>16 | C 07 C 1/04<br>C 07 C 29/15<br>B 01 J 27/04<br>C 01 G 39/06 |
| X | WO-A-8 503 073 (UNION CARBIDE)<br><br>* Claims *<br><br>--- | 1,2,5,<br>6,11,<br>13,16 | |
| A | US-A-4 528 089 (PECORARO et al.)<br>* Claims *<br><br>--- | 13,14,<br>16 | |
| A | THERMOCHIMICA ACTA, vol. 81, 1984, pages 281-296, Elsevier Science Publishers B.V., Amsterdam, NL; Q. WU et al.: "Characterization of unsupported CoMo sulfide catalysts and their precursors by temperature-programmed reactions"<br><br>--- | | TECHNICAL FIELDS SEARCHED (Int. Cl.4)<br><br>C 07 C 1/00<br>C 07 C 29/00 |
| A,D | JOURNAL OF CATALYSIS, vol. 95, 1985, pages 249-259, Academic Press, Inc., New York, US; K. RAMANATHAN et al.: "Characterization of tungsten sulfide catalysts"<br><br>--- -/- | | |

The present search report has been drawn up for all claims

| Place of search | Date of completion of the search | Examiner |
|---|---|---|
| THE HAGUE | 12-11-1986 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

## DOCUMENTS CONSIDERED TO BE RELEVANT

Page  2

| Category | Citation of document with indication, where appropriate, of relevant passages | Relevant to claim | CLASSIFICATION OF THE APPLICATION (Int. Cl.4) |
|---|---|---|---|
| A,P | JOURNAL OF CATALYSIS, vol. 95, 1985, pages 455-464, Academic Press, Inc., New York, US; D.G. KALTHOD et al.: "Studies of molybdenum sulfide catalysts: Effects of pretreatment on sintering, stoichiometry, and oxygen chemisorption" <br><br> --- | | |
| D,X | JOURNAL OF INORGANIC AND NUCLEAR CHEMISTRY, vol. 35, 1973, pages 1895-1904, Pergamon Press., Oxford, GB; T.P. PRASAD et al.: "Thermal decomposition of $(NH_4)_2MoO_2S_2$, $(NH_4)_2MoS_4$, $(NH_4)_2WO_2S_2$ and $(NH_4)_2WS_4$" <br><br> ----- | 13,14, 16 | |

TECHNICAL FIELDS SEARCHED (Int. Cl.4)

The present search report has been drawn up for all claims

| Place of search | Date of completion of the search | Examiner |
|---|---|---|
| THE HAGUE | 12-11-1986 | VAN GEYT J.J.A. |